(19) Europäisches Patentamt / European Patent Office / Office européen des brevets

(11) **EP 3 060 300 B1**

(12) **FASCICULE DE BREVET EUROPEEN**

(45) Date de publication et mention
de la délivrance du brevet:
**05.12.2018 Bulletin 2018/49**

(21) Numéro de dépôt: **14801947.4**

(22) Date de dépôt: **27.10.2014**

(51) Int Cl.:
*A61N 5/06* (2006.01)        *A61F 7/00* (2006.01)

(86) Numéro de dépôt international:
**PCT/EP2014/073032**

(87) Numéro de publication internationale:
**WO 2015/059313 (30.04.2015 Gazette 2015/17)**

(54) **DISPOSITIF D'IRRADIATION À INFRAROUGE**

INFRAROTSTRAHLUNGSVORRICHTUNG

INFRARED RADIATION DEVICE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **25.10.2013 FR 1360481**

(43) Date de publication de la demande:
**31.08.2016 Bulletin 2016/35**

(73) Titulaire: **Vital Tech**
**92100 Boulogne Billancourt (FR)**

(72) Inventeurs:
• **FAUCHON, Eric**
**92100 Boulogne Billancourt (FR)**
• **GAVSEVITCH, Alexandra**
**92100 Boulogne Billancourt (FR)**

(74) Mandataire: **Debay, Yves**
**Cabinet Debay**
**126, Elysee 2**
**78170 La Celle Saint Cloud (FR)**

(56) Documents cités:
WO-A2-2012/093347        CA-A1- 2 450 633
GB-A- 2 258 474            US-A- 6 004 344
US-A1- 2002 183 814      US-A1- 2006 226 378

EP 3 060 300 B1

## Description

### DOMAINE TECHNIQUE DE L'INVENTION

La présente invention se rapporte au domaine des traitements par radiation, plus particulièrement un dispositif d'irradiation par infrarouge.

### ARRIÈRE-PLAN TECHNOLOGIQUE DE L'INVENTION

L'infrathérapie ou l'utilisation d'ondes infrarouges en thérapie permet une résonance du tissu musculaire augmentant les fonctions cellulaires et améliorant la circulation sanguine, le système cardiovasculaire ainsi que le système immunitaire. L'énergie radiante d'un dispositif d'irradiation à infrarouge fonctionne par fréquence de résonance sur la membrane cellulaire procurant une désintoxication en profondeur des couches supérieures du corps humain soumis à divers agents polluants. Par exemple, cette énergie radiante peut avoir un effet bénéfique sur le bien-être, sur la santé ou sur les performances sportives d'un utilisateur (9) d'un dispositif d'irradiation à infrarouge.

Il est connu dans l'art antérieur des dispositifs d'irradiation à infrarouge comprenant un support destiné à recevoir un utilisateur et une partie recouvrante propre à recouvrir l'utilisateur et comprenant une couche chauffante comportant des moyens d'émission infrarouge pour irradier l'utilisateur. Un tel dispositif est décrit par exemple dans US 2002/183814 A1. Certains de ces dispositifs possèdent souvent des moyens d'émission infrarouge comprenant des plaques de carbone qui sont fiables et fournissent un rayonnement dans des longueurs d'ondes avantageuses. Cependant, ces dispositifs ont généralement le désavantage de ne pas garantir une longévité et/ou fiabilité du dispositif. En particulier, les dispositifs destinés à un usage professionnel nécessitent de pouvoir fonctionner souvent et longtemps. Il est alors particulièrement important de garantir une bonne fiabilité du dispositif dans le temps.

### DESCRIPTION GÉNÉRALE DE L'INVENTION

L'invention est définie dans les revendications attenantes. La présente invention a pour objet d'obtenir un système d'irradiation par infrarouge améliorant la longévité et/ou la fiabilité du dispositif . En particulier, l'invention permet de réguler la température et de garantir un fonctionnement optimal au cours du temps, pendant chaque séance et/ou au cours de la durée de vie optimisée du dispositif.

À cet effet, l'invention concerne un dispostif d'irradiation à infrarouge comprenant un support apte à recevoir un utilisateur et une partie recouvrante apte à recouvrir l'utilisateur, la partie recouvrante comprenant au moins une couche chauffante apte à émettre un rayonnement infrarouge dans une plage de longueurs d'onde déterminée vers au moins une partie du volume situé entre la face interne de la couche chauffante et le support, ladite couche chauffante étant régulée par au moins un contrôleur comprenant des moyens d'alimentation fournissant la puissance nécessaire à l'émission d'infrarouge pendant une séance d'irradiation, le dispositif étant caractérisé en ce qu'il comprend en outre au moins un programmateur déterminant les paramètres de la séance d'irradiation, en définissant des pulsations au cours de la séance ; ces pulsations contrôlant, d'une part, l'alimentation de ladite couche chauffante à des fréquences variables, et d'autre part, au moins un contacteur du dispositif, par exemple un relais statique synchrone ou un autre type de contacteur qui délivre, à ladite couche chauffante, la puissance fournie par les moyens d'alimentation au cours des pulsations.

Selon une autre particularité, ledit relais statique synchrone ne délivre aucun courant à ladite couche chauffante en dehors des créneaux temporels définis par le programmateur.

Selon une autre particularité, le programmateur calcule le temps auquel les créneaux temporels doivent être délivrés, ainsi que leur durée, en fonction de la quantité d'énergie à fournir, et donc de la température demandée.

Selon une autre particularité, le programmateur comporte une mémoire stockant une pluralité de programmes prédéfinis correspondant à divers types de séances d'irradiation et définissant la durée de la séance d'irradiation et/ou la ou les température(s) à atteindre au cours de la séance.

Selon une autre particularité, le programmateur calcule les créneaux temporels en fonction d'informations de température provenant d'au moins une sonde ou capteur du dispositif.

Selon une autre particularité, la dite au moins une sonde ou capteur est au moins une sonde de température permettant au programmateur d'ajuster les pulses en fonction de la température obtenue dans le dispositif.

Selon une autre particularité, le dispositif est muni de moyens de sécurité électrique pour protéger l'utilisateur d'éventuels problèmes dans les circuits électriques du dispositif.

Selon une autre particularité, la partie recouvrante comprend en outre une couche réflectrice de rayonnement infrarouge.

Selon une autre particularité, le support comprend une deuxième couche chauffante apte à émettre un rayonnement dans au moins une partie du volume entre la face interne de la première couche chauffante et le support.

Selon une autre particularité, une extrémité, selon l'axe longitudinal, de la partie recouvrante comprend une troisième couche chauffante fermant la partie recouvrante.

Selon une autre particularité, la partie recouvrante comprend au moins deux portions, la deuxième portion de la partie recouvrante étant apte à coulisser par rapport à la première portion parallèlement à l'axe longitudinal de sorte à s'escamoter dans ou sur la première portion.

Selon une autre particularité, le câblage d'alimentation de la première couche chauffante comporte un guide pour permettre le coulissement des deux portions sans coincer et/ou endommager le câblage.

Selon une autre particularité, la partie recouvrante comprend une couche extérieure protégeant l'intérieur de la partie recouvrante.

Selon une autre particularité, les couches chauffantes comprennent au moins une plaque en carbone.

Selon une autre particularité, la couche réflectrice comprend au moins une couche en aluminium.

Selon une autre particularité, la couche extérieure est en bois.

Selon une autre particularité, le support est en bois.

Selon une autre particularité, la surface interne de la partie recouvrante est recouverte d'un textile.

Selon une autre particularité, le dispositif d'irradiation comprend un système de relèvement de la partie recouvrante, la première portion ou la deuxième portion de la partie recouvrante étant fixée sur au moins le système de relèvement de la partie recouvrante, le système de relèvement de la partie recouvrante comprenant au moins un vérin de poussée dont une extrémité est fixée sur le support ou sur les pieds du support et l'autre extrémité est fixée sur la première ou la deuxième portion de la partie recouvrante de manière à ce que le vérin puisse exercer une poussée tendant à lever une extrémité de la partie recouvrante, l'autre extrémité de la partie recouvrante étant fixée sur le support par l'intermédiaire d'une articulation autorisant le fait que l'axe longitudinal de la partie recouvrante puisse faire un angle non nul avec le plan du support.

Selon une autre particularité, le câblage d'alimentation de la couche chauffante passe à proximité de l'articulation de la partie recouvrante pour en faciliter le pivotement sans coincer et/ou endommager le câblage.

L'invention, avec ses caractéristiques et avantages, ressortira plus clairement à la lecture de la description faite en référence aux dessins annexés dans lesquels :

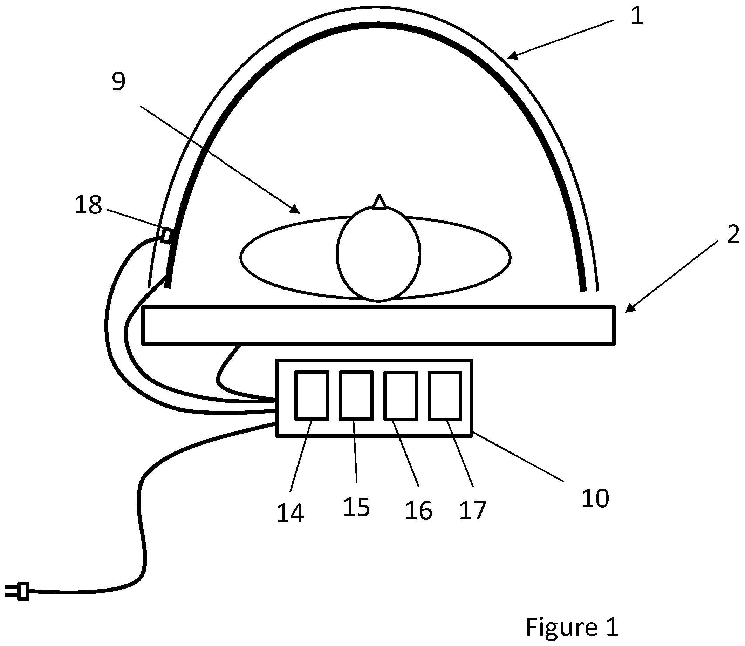

La figure 1 représente une vue du dispositif selon un axe de vue parallèle à l'axe longitudinal du dispositif.

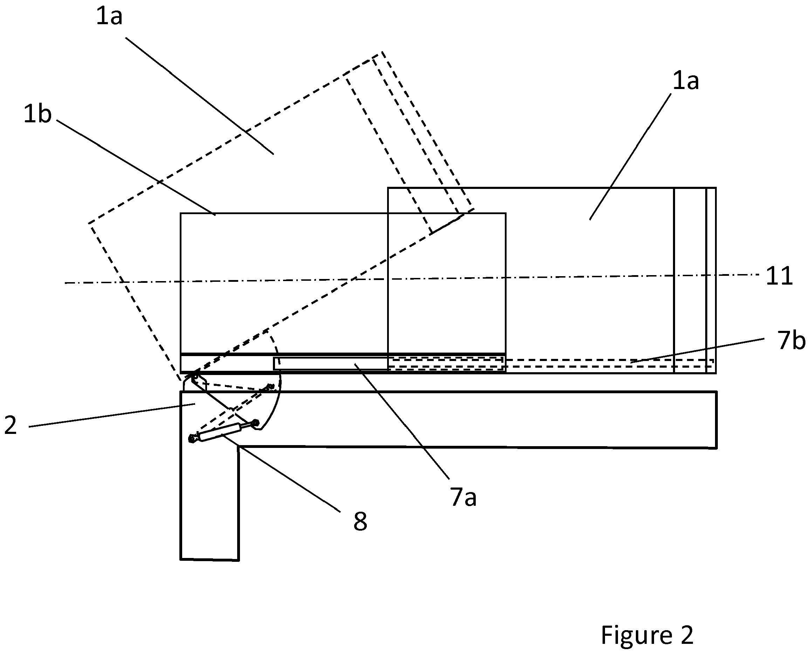

La figure 2 représente une vue de profil du dispositif selon deux positions de la partie recouvrante.

La figure 3 représente un schéma de câblage du dispositif avec son contrôleur.

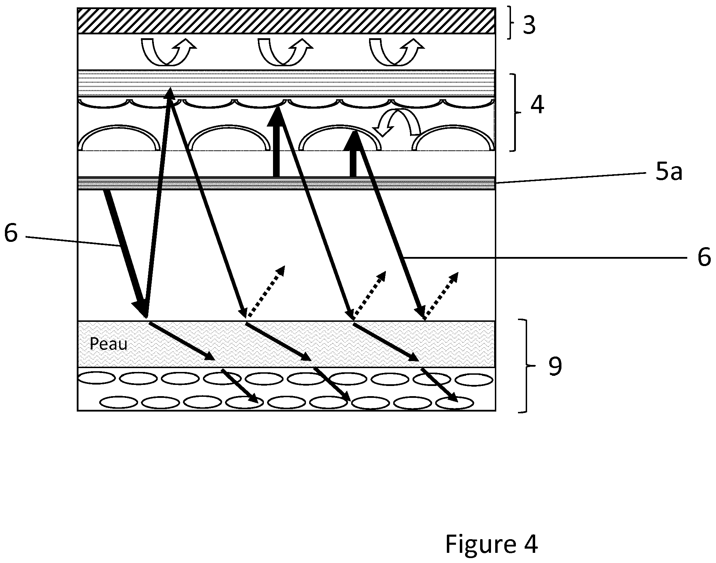

La figure 4 représente schématiquement le comportement des rayonnements infrarouge entre la partie recouvrante et l'utilisateur du dispositif.

La figure 5 représente une vue éclatée en perspective du dispositif.

DESCRIPTION DES MODES DE RÉALISATION PRÉFÉRÉS DE L'INVENTION

La suite de la description fera référence aux figures citées ci-dessus.

L'invention concerne un dispositif d'irradiation à infrarouge. Ce dispositif est parfois autrement appelé sauna individuel. Dans la suite de la description, le dispositif pourra être appelé dispositif d'irradiation ou simplement dispositif.

À la différence d'un sauna traditionnel qui réchauffe un corps indirectement en chauffant l'air ambiant, le dispositif d'irradiation à infrarouge permet de réchauffer le corps directement par la chaleur produite par le rayonnement (6) infrarouge. La température atteinte par le dispositif peut avantageusement se situer dans une plage allant de la température ambiante à environ 80°C, mais peut s'étaler jusqu'aux températures atteintes par les saunas traditionnels allant jusqu'à 105°C. La température ambiante est généralement une température égale à 20°C ou légèrement inférieure à 20°C, mais la température ambiante peut aller jusqu'à 35°C ou 40°C. Le fait de réguler la température en-dessous de valeurs excessives permet avantageusement, outre le confort du patient, d'améliorer la durée de vie du dispositif.

De plus, le dispositif d'irradiation comporte des moyens d'émission infrarouges (5a, 5b, 5c) qui sont de préférence formés par des plaques en carbone et qui sont alimentées, via le secteur, pour émettre les infrarouges dans une plage de longueur d'onde déterminée. Ces moyens d'émission infrarouges sont régulés par un contrôleur (10) comprenant des moyens d'alimentation (16) fournissant la puissance nécessaire à l'émission d'infrarouge et un programmateur (15) déterminant les paramètres d'une séance d'irradiation, en définissant des créneaux temporels (pulsations ou puises) au cours de la séance, ces créneaux temporels contrôlant au moins un relais statique synchrone ou un autre type de contacteur délivrant, aux moyens d'émission infrarouges (5a, 5b, 5c), la puissance fournie par les moyens d'alimentation (16) au cours des créneaux temporels. Dans certains modes de réalisation préférés, le contacteur est un relais statique synchrone (14) qui ne délivre aucun courant aux moyens d'émission infrarouges (5a, 5b, 5c) en dehors des créneaux temporels définis par le programmateur (15).

Le programmateur (15) définit divers paramètres relatifs au contrôle des moyens d'émission infrarouges (5a, 5b, 5c) pendant au moins une séance d'irradiation. De préférence, le programmateur (15) calcule le temps auquel les créneaux temporels doivent être délivrés au cours de la séance, ainsi que leur durée, en fonction de la quantité d'énergie à fournir, et donc de la température demandée. Cette température est généralement déterminée en fonction de la longueur d'onde prévue pour les moyens d'émission et en fonction des paramètres physiques du dispositif (dimensions, isolation etc.). De plus, ce programmateur peut stocker en mémoire une pluralité de programmes prédéfinis correspondant à divers types de séances d'irradiation. Ces programmes définissent des paramètres de la séance, par exemple tels que la durée de la séance d'irradiation, la ou les température(s) à atteindre au cours de la séance et peuvent tenir compte d'autres informations comme par exemple la taille, le poids ou l'âge de l'utilisateur à irradier, la température ambiante de la pièce et/ou la température à l'intérieur du dispositif. Ainsi, au cours d'une séance d'irradiation, le contrôleur (10) alimente les moyens d'émission infrarouges (5a, 5b, 5c) pendant au moins une période de temps déterminée par le programmateur (15), ou plusieurs périodes de temps d'irradiation si le programme prévoit des pauses au cours de la séance. Pendant chaque période de temps d'irradiation, le programmateur (15) délivre une pluralité de pulsations (pulses) grâce aux créneaux temporels définis dans le (ou les) programme(s) pour contrôler les moyens d'émission infrarouges (5a, 5b, 5c). Ces créneaux temporels ont une durée et une récurrence déterminées pour optimiser la régulation de l'irradiation et obtenir ainsi de manière plus fiable la température définie dans le (ou les) programme(s). En effet, en procédant ainsi avec des pulsations au lieu de chauffer en continu pendant une période de temps d'irradiation donnée, l'irradiation est mieux contrôlée au niveau des moyens d'émission infrarouges (5a, 5b, 5c) qui sont alors plus aptes à fournir la température souhaitée, alors qu'avec une alimentation continue, ils chaufferaient trop ou trop peu et trop vite ou trop lentement (selon la puissance électrique et la durée d'alimentation fournies). Les dispositifs d'irradiation infrarouge de l'art antérieur sont connus pour présenter de tels problèmes puisqu'ils comportent simplement un minuteur définissant une période de temps d'irradiation pendant laquelle les moyens d'émission infrarouges (5a, 5b, 5c) sont alimentés sans arrêt. A l'inverse, le courant pulsé obtenu grâce au contrôleur (10) permet de réguler, à des fréquences variables (selon la durée des puises, par exemple de l'ordre du Hz jusqu'au kHz), l'alimentation des moyens d'émission infrarouges (5a, 5b, 5c) et d'obtenir ainsi un contrôle très fin de la température fournie.

Dans certains modes de réalisation, le programmateur (15) calcule les pulses en fonction d'informations de température provenant d'au moins une sonde (18) du dispositif. Ainsi, le dispositif est muni d'une ou plusieurs sonde(s) (18, 18a, 18b, 18c) de température permettant au programmateur d'ajuster les pulses en fonction de la température obtenue dans le dispositif. D'autres types de sondes ou capteurs peuvent être utilisés (de préférence en complément des sondes de température), comme par exemple des capteurs de poids permettant au programmateur d'ajuster les pulses automatiquement ou ajuster le type de programme prédéfini à utiliser en fonction du poids de l'utilisateur. Des sondes d'hydrométrie peuvent également être utilisées pour permettre d'affiner la régulation par le programmateur.

Cet agencement présente l'avantage de n'alimenter les moyens d'émission infrarouge que lorsque c'est nécessaire (pendant les pulses définis par le programmateur) et de permettre une régulation fine de la température. Dans certains modes de réalisation préférés, grâce au(x) programmateur(s) (15) définissant les pulses et grâce au(x) relais statique(s) synchrone(s) (14) qui coupe(nt) la puissance à la tension nulle en dehors des puises, les moyens d'émission infrarouge (5a, 5b, 5c) ne sont sollicités que lorsque c'est nécessaire, ce qui améliore la longévité du dispositif et en particulier des moyens d'émission infrarouge (de préférence formés par des plaques de carbone). D'autres types de contacteurs peuvent être utilisés, mais on préfère les relais statiques synchrones pour préserver les moyens d'émission infrarouge. De plus, ces moyens d'émission infrarouge sont sollicités, par le programmateur, pour atteindre et maintenir une température définie et la régulation fine obtenue par les pulsations du programmateur améliore la fiabilité du dispositif comme détaillé ci-dessus. Enfin, les relais statiques synchrones, qui épargnent les moyens d'émission infrarouge par le fait qu'ils coupent la tension à 0 en l'absence de pulse du programmateur, présentent également les avantages de limiter les perturbations, de produire moins de parasites et d'émission rayonnantes, ce qui peut être particulièrement avantageux, notamment si la fréquence des pulses (déterminés par le programmateur) est élevée. Ces avantages permettent également de prolonger la durée de vie du dispositif dans son ensemble.

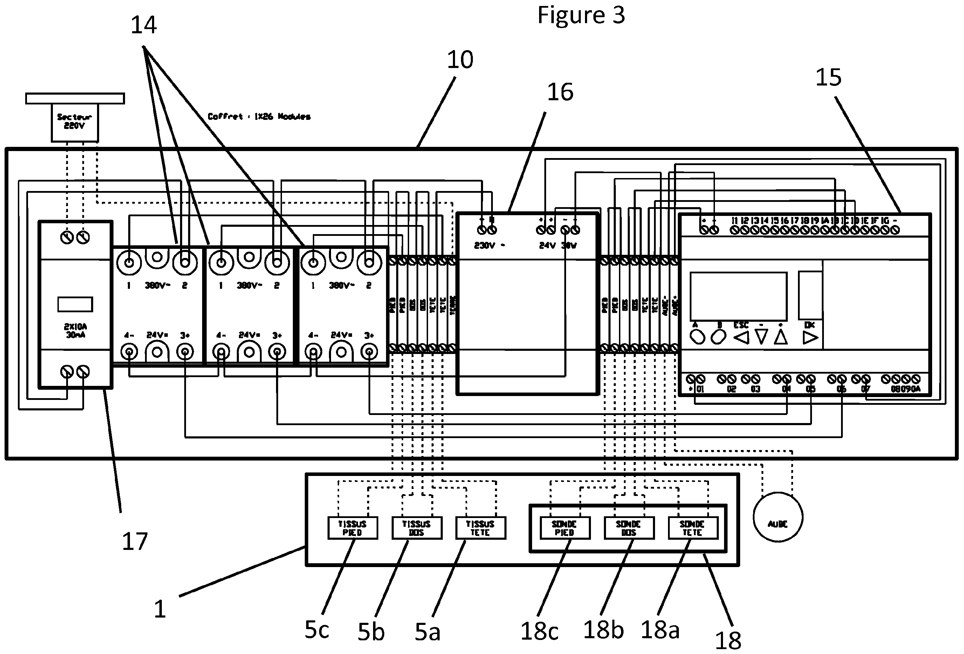

On prévoit en général des moyens de sécurité électrique (17), comme par exemple un disjoncteur différentiel (17), pour protéger l'utilisateur d'éventuels problèmes dans les circuits électriques du dispositif, comme par exemple représenté sur la figure 3.

La figure 3 représente, de manière illustrative et nullement limitative, un schéma de câblage d'un dispositif selon des modes de réalisation préférés de l'invention. Le secteur délivre un courant alternatif à un transformateur (16) apte à fournir la puissance aux moyens d'émission infrarouges. Ce transformateur alimente (en 24V dans l'exemple représenté) au moins un programmateur (15) qui pilote (par des pulses de courant, en 24V dans l'exemple représenté) au moins relais statique synchrone (14). Au cours des puises, le relais statique synchrone délivre aux moyens d'émission infrarouge la puissance permettant l'émission d'infrarouge nécessaire pour atteindre la température définie. En l'absence de pulse, il coupe la tension à la valeur nulle et les moyens d'émission infrarouge sont alors protégés et aucune émission de pertubations, parasites ou émissions rayonnantes ne vient raccourcir la durée de vie du dispositif.

Dans certains modes de réalisation, le dispositif comprend un moyen de réglage permettant de régler l'intensité de rayonnement des moyens d'émission infrarouge. Ces moyens de réglages permettent de commander le contrôleur. Cette commande varie naturellement en fonction des modes de réalisation choisis et on comprend qu'on peut, par exemple, soit simplement pour définir une température soit pour sélectionner un programme donné, soit pour saisir un ensemble d'informations nécessaires aux calculs des pulses par le programmateur.

L'exemple illustratif et non limitatif de la figure 3 représente 3 relais statiques synchrones (14) qui alimente 3 paires de moyens d'émission infrarouges (désignés par le terme « tissus » sur la figure 3), ce qui correspond en fait aux agencements illustratifs et non limitatifs du dispositif représenté sur les autres figures. De même, 3 paires de sondes (18a, 18b, 18c) sont représentées pour fournir au programmateur les températures mesurées dans les portions du dispositif qui correspondent à chaque paire de moyens d'émission. On comprendra que l'on peut s'éloigner de cet exemple tout en restant dans le cadre de l'invention, par exemple en fournissant moins de sondes qu'il n'y a de moyens d'émission. De plus, on comprend bien entendu que ces nombres pourront varier, mais qu'on prévoit de préférence autant de relais qu'il y aura de moyens d'émission à contrôler indépendamment. De même, on ne représente qu'un transformateur (16), qu'un programmateur (15) et qu'un disjoncteur (17) mais si le dispositif dépassait les capacités de chacun de ces composants, on les multiplierait pour obtenir les mêmes résultats.

Dans la suite de la description faite en référence aux figures, on désigne les moyens d'émission infrarouges par les termes de couche chauffante ou de plaque de carbone.

Le dispositif d'irradiation à infrarouge comprend un support (2). Ce support (2) permet de recevoir un utilisateur (9), par exemple, allongé. Le support (2) est surmonté de la partie (1) recouvrante qui permet de recouvrir et border les côtés de l'utilisateur (9) qui est, par exemple, allongé sur le support (2). Le support (2) et la partie (1) recouvrante sont disposés selon un axe longitudinal du dispositif.

La partie (1) recouvrante comprend au moins une couche chauffante. Dans certains modes de réalisation, cette couche comporte au moins une plaque en carbone. On désigne donc parfois, de façon non limitative, dans la présente description les couches chauffantes par le terme « plaques en carbone ». Les plaques en carbone sont produites généralement à partir d'un tissu polymère supraconducteur composé de carbone, de silice et de graphite. Le tissu est de préférence isolé entre deux feuilles de fibre de verre stratifié époxy et laminées à haute température. La forte densité et la répartition régulière du carbone permet une émission régulière du rayonnement (6) infrarouge sur toute sa surface. Une feuille en fibre de verre comprend généralement, sur deux bords opposés de la plaque en carbone, une matière conductrice d'électricité telle que le cuivre en contact avec le tissu et alimentée en électricité par l'intermédiaire d'un contrôleur (10). Par exemple, les deux bords ont deux bandes en cuivre alimentant le tissu.

La partie (1) recouvrante comprend au moins une couche (5a, 5b) chauffante. Cette couche (5a, 5b) chauffante est apte à émettre un rayonnement (6) infrarouge dans au moins une partie du volume situé entre la face interne de la couche (5a, 5b) chauffante et le support (2).

Le rayonnement (6) infrarouge a comme particularité une émission d'ondes de la première couche (5a) chauffante qui provoque une élévation de température de l'utilisateur (9) récepteur des ondes émises.

De façon non limitative, les ondes émises ont des longueurs d'onde comprises entre 0,76 μm et 10 μm. L'avantage de ce mode de chauffage réside dans le fait que les infrarouges ne sont pas ou peu absorbés par l'air, alors que les solides et les liquides les absorbent facilement. Les longueurs d'onde sont inversement proportionnelles à la température de leur émetteur. D'après la loi de Wien, plus la température de l'émetteur est élevée, plus la longueur d'onde est courte.

L'absorption par les tissus biologiques des photons des ondes infrarouges modifie l'état de vibration ou de rotation moléculaire. En raison de leur faible énergie, les photons infrarouges ne peuvent pas produire d'ionisation, ni de réactions photochimiques. L'irradiation infrarouge est globalement perçue comme une source de chaleur procurant une détente et un bien-être, voire des avantages supplémentaires comme décrits dans la présente demande.

Dans certains modes de réalisation, les couches chauffantes du dispositif émettent un rayonnement (6) infrarouge long dans la plage de longueur d'onde comprise entre 6 μm à 19 μm. La peau humaine est une matière complexe considérée comme un corps gris dont l'émissivité est proche de celle d'un corps noir. Elle dissipe sa chaleur en partie par radiation. Pour un corps noir, la loi de Stefan-Boltzmann intègre la notion d'émissivité qui représente un rapport entre l'énergie rayonnée par une surface subissant un flux incident et l'énergie que rayonnerait un corps noir à la même température. L'émissivité peut varier en fonction de la longueur d'onde. La loi de déplacement de Wien est obtenue par la dérivation de la loi de Planck. Elle donne la longueur d'onde correspondant au maximum de rayonnement spectral d'un corps noir en fonction de sa température. À partir de cette loi, on remarque que la peau à une température de 35°C émet un maximum d'énergie à une longueur d'onde de 9,4 μm. À une température de 37°C, la peau émet un maximum d'énergie à une longueur d'onde de 9,3 μm. À une température de 38,4°C, la peau émet un maximum d'énergie à une longueur d'onde de 9,3 μm.

Par conséquent, de préférence, les couches chauffantes du dispositif d'irradiation à infrarouge émettent à une longueur comprise entre 8,7 μm et 9,5 μm. Encore de préférence, elles émettent à une longueur d'onde de 9,3 μm.

De plus, il est connu que certains corps, notamment comprenant du carbone et du silicium peuvent émettre un rayonnement infrarouge qui se propage dans une direction privilégiée perpendiculaire à sa surface, par exemple comme cela est démontré dans la thèse de doctorat de Martine Laroche, (Rôle des ondes de surface dans la modification des propriétés radiatives de matériaux microstructurés. Application à la conception de sources infrarouges et à l'effet thermophotovoltaïque. École centrale, Paris, 2005). Ainsi, la ou les couches chauffantes, comprenant de préférence du carbone, du silicium et du graphite, utilisés dans certains modes de réalisation de la présente demande, émettent un rayonnement infrarouge dont la direction privilégiée est perpendiculaire à leur surface en tous points. Ce rayonnement directionnel peut être ressenti par le fait que la ou les couches chauffent l'espace face à elle et non pas à côté. Ceci a pour avantage de chauffer uniquement l'espace à l'intérieur du dispositif et de limiter le réchauffement ambiant dans la pièce où se trouve le dispositif. De plus, divers modes de réalisation tirent avantage de ce rayonnement directionnel en utilisant un profil particulier.

En effet, dans certains modes de réalisation, la section de la première couche (5a) chauffante transversalement à l'axe longitudinal suit avantageusement une forme représentative d'une courbe parabolique. On désigne ici cette forme de la section transversale par le terme profil et en particulier de profil parabolique. De plus, comme le dispositif peut comporter plusieurs couches chauffantes, on désigne ici cette couche chauffante à profil parabolique par le terme « première couche chauffante » mais ce terme ne doit pas être interprété de façon limitative car il désigne en fait la partie rayonnante du dôme au-dessus de l'utilisateur et composé d'une ou plusieurs couches chauffantes. Le profil parabolique de la première couche (5a, 5b) chauffante permet d'obtenir une zone de convergence du rayonnement (6) infrarouge mieux répartie sur l'ensemble du corps de l'utilisateur (9). Par exemple comme représenté sur la figure 3, le rayonnement infrarouge est émis selon une direction privilégiée perpendiculaire à la surface de la couche chauffante en chaque point de la couche chauffante. La combinaison du profil parabolique avec cette direction privilégiée perpendiculaire permet une répartition du rayonnement de manière homogène. Dans la zone de convergence, la valeur de température générée en Kelvin peut être multipliée par un facteur de 1,4 pour deux rayons qui se croisent.

Dans certains modes de réalisation, bien qu'ils ne soient pas préférés, la première couche (5a, 5b) chauffante peut avoir un profil de forme semi-circulaire, mais le rayonnement (6) infrarouge la zone de convergence se réduit en un seul point qui serait le centre du cercle de la forme semi-circulaire. Ceci a pour conséquence une moins bonne répartition du rayonnement (6) infrarouge qui est focalisée sur un axe parallèle à l'axe longitudinal du dispositif, tandis qu'avec un profil parabolique, la répartition peut avantageusement être ajustée pour suivre au moins approximativement la forme extérieure du corps de l'utilisateur présent à l'intérieur du dispositif. Ainsi, outre le confort de l'utilisateur, une telle répartition du chauffage permet au dispositif de trouver des applications en phlébologie, telles que par exemple la réduction de varices, ce qui serait peu probable pour un dispositif de l'art antérieur.

Dans certains modes de réalisation, la courbe parabolique a pour fonction y = x$^2$- x -1. On notera que le nombre d'or $\dfrac{1+\sqrt{5}}{2}$ est la solution positive pour x$^2$

- x -1 = 0, où x représente les coordonnées selon l'axe des abscisses parallèle au support (2) du dispositif et y représente les coordonnées selon l'axe des ordonnées perpendiculaire au support (2) du dispositif avec l'axe des ordonnées se dirigeant vers le sol.

Dans certains modes de réalisation, le dispositif comporte, par exemple au niveau du contrôleur (10), au moins un moyen d'affichage permettant d'afficher la longueur d'onde émise par la couche chauffante alimentée par l'intermédiaire du contrôleur (10). Un tel affichage peut être réalisé également sur un moyen d'affichage (13) du dispositif. En effet, dans certains modes de réalisation, le dispositif comprend un moyen d'affichage (13) permettant d'afficher des informations concernant le fonctionnement du dispositif telles que la température produit par les couches chauffantes, le temps d'utilisation du dispositif ou d'autres paramètres utiles pour le fonctionnement du dispositif.

Dans certains modes de réalisation, la partie (1) recouvrante du dispositif d'irradiation à infrarouge comprend aussi une couche (4) réflectrice de rayonnement (6) infrarouge sur la face externe de la couche chauffante. De préférence, le profil de la couche (4) réflectrice suit le profil de la couche (5a, 5b) chauffante. Cette couche (4) réflectrice permet de réfléchir le rayonnement (6) infrarouge qui n'a pas été absorbé par le corps de l'utilisateur (9) pour être renvoyé vers le corps de l'utilisateur (9). Ceci permet une économie d'énergie nécessaire pour émettre le rayonnement (6) infrarouge. Ceci permet également une sécurité du dispositif d'irradiation à infrarouge car la surface externe de la partie (1) recouvrante reste froide.

Dans une configuration, la couche (4) réflectrice comprend au moins un film ou une plaque en aluminium. Elle peut, en complément ou en alternative également comporter au moins un film ou une plaque contenant du cobalt, particulièrement efficace pour refléter les infrarouges.

Dans certaines configurations de la couche (4) réflectrice, elle comprend plusieurs composants. Dans une configuration préférée, les composants comprennent six films réflecteurs, deux couches en ouate et six couches en mousses.

Dans certains modes de réalisation, la partie (1) recouvrante du dispositif comprend une couche (3) extérieure protégeant l'intérieur de la partie (1) recouvrante. Dans certains modes de réalisation préférés, cette couche (3) est rigide. Dans certains modes de réalisation, cette couche (3) extérieure est en matière plastique ou en métal ou en bois. Dans certains modes de réalisation, la couche (3) extérieure peut être réalisée en fibres de verre ou en fibres de carbone ou en toute fibre moulable. Dans certains modes de réalisation, la couche (3) extérieure peut être réalisée par une combinaison des matériaux énumérés ci-avant.

La couche (4) réflectrice du dispositif est disposée entre la couche (3) extérieure et la couche chauffante. La couche chauffante est ainsi sans contact avec la couche (3) extérieure du dispositif, ce qui permet de limiter fortement les transferts d'énergie par conduction et procure une bonne efficacité thermique par rapport à l'art antérieur.

Dans certains modes de réalisation, la couche (3) extérieure a un profil de forme parabolique qui suit le profil de la couche chauffante. Par exemple, la couche (4) réflectrice, la couche (3) extérieure et la première couche (5a) chauffante sont fixes les unes par rapport aux autres. De préférence, elles sont fixées par des moyens de fixation supportant la chaleur et n'entraîne pas l'émission de vapeurs, voire permettant une isolation entre au moins 2 de ces 3 couches, par exemple grâce à un espace (e.g., une lame d'air) entre au moins 2 de ces couches, en particulier entre la couche (4) réflectrice et la couche extérieure (3). Les moyens de fixation sont par exemple des gorges dans lesquelles sont emboîtées les extrémités des différentes couches. Ces gorges sont, par exemple, disposés sur au moins deux bords situés aux extrémités opposées de la couche extérieure et dans lesquelles sont insérées les couches chauffante et réflectrice.

Dans certains modes de réalisation, le support (2) comprend une deuxième couche (non représentée) chauffante apte à émettre un rayonnement dans au moins une partie du volume entre la face interne de la couche chauffante et le support (2). Par exemple, la deuxième couche chauffante est intégrée dans un matelas, par exemple en matériau amortisseur ou confortable, posé sur le support (2).

Dans certains modes de réalisation, une première extrémité de la partie recouvrante selon l'axe longitudinal (11) est fermée. Dans certains de ces modes de réalisation, cette première extrémité de la partie (1) recouvrante comprend aussi une troisième couche (5c) chauffante qui chauffe la région des pieds de l'utilisateur.

Dans certains modes de réalisation, la deuxième extrémité est ouverte.

Dans certains modes de réalisation, la deuxième extrémité de la partie recouvrante comprend des moyens de fermeture amovible et/ou partielle de la deuxième extrémité. Dans certains de ces modes de réalisation, de tels moyens de fermeture amovible et/ou partielle sont avantageusement formés par un porte-serviette (12). Le porte-serviette est formé, par exemple, par une bande en textile élastique fixée sur la partie recouvrante de telle manière que la partie entre les deux extrémités de la bande peut être soulevée pour pouvoir introduire une serviette. Cette serviette permet alors de refermer la deuxième extrémité de la partie recouvrante.

Dans certains modes de réalisation, la surface interne de la partie recouvrante est recouverte d'un matériau évitant à l'utilisateur de pas être en contact direct avec la couche chauffante, comme par exemple un textile.

Dans certains modes de réalisation, la surface du support destinée à être en contact avec l'utilisateur est recouverte d'un matériau évitant à l'utilisateur de pas être en contact direct avec le support et l'éventuelle couche chauffante qu'il peut contenir. Un tel matériau peut être en textile et on peut lui associer un matériau amortisseur pour le confort du patient. Comme l'utilisateur transpire dans le dispositif, on préfère généralement que ce matériau soit imperméable ou qu'il disposé de manière amovible sur la surface.

Dans certains modes de réalisation, la partie (1) recouvrante comprend au moins deux portions dont une première portion (1a) et une deuxième portion (1b). La première portion (1a) peut s'escamoter sur ou dans la deuxième portion (1b) par coulissement de la première portion (1a) selon l'axe (11) longitudinal de la partie (1) recouvrante. Ainsi, par exemple, lorsque la première portion (1a) s'escamote sur la deuxième portion (1b), la première portion (1a) recouvre la deuxième portion (1b). On préfère en général que la portion qui recouvre l'autre soit celle qui correspond à l'extrémité près de laquelle l'utilisateur aura sa tête, en l'occurrence la première portion (1a) sur les figures illustratives et non limitatives.

Dans certains modes de réalisation, le coulissement est permis par un système (7) de coulissement. Selon une configuration particulière, un tel système de coulissement est obtenu par des glissières telles que des mécanismes de tiroir. Dans une autre configuration,, un tel système de coulissement peut comprendre au moins un coulisseau fixé sur la première portion (1a) de la partie (1) recouvrante. Le ou les coulisseaux sont fixés parallèle à l'axe (11) longitudinal de la partie (1) recouvrante à l'extrémité des branches de la forme parabolique. Le ou les coulisseaux sont aptes à se déplacer chacun dans une coulisse fixée sur le support (2) selon un axe (11) longitudinal de la partie (1) recouvrante. Dans une autre configuration, le coulisseau (7a) est fixé sur la surface extérieure de la deuxième portion (1b) parallèlement à l'axe (11) longitudinal de la partie (1) recouvrante au niveau des extrémités des branches de la forme parabolique. Les coulisses (7b) dans lesquelles les coulisseaux (7a) peuvent coulisser sont fixées sur la surface intérieure de la première portion (1a) parallèlement à l'axe (11) longitudinal de la partie (1) recouvrante au niveau des extrémités de la forme parabolique.

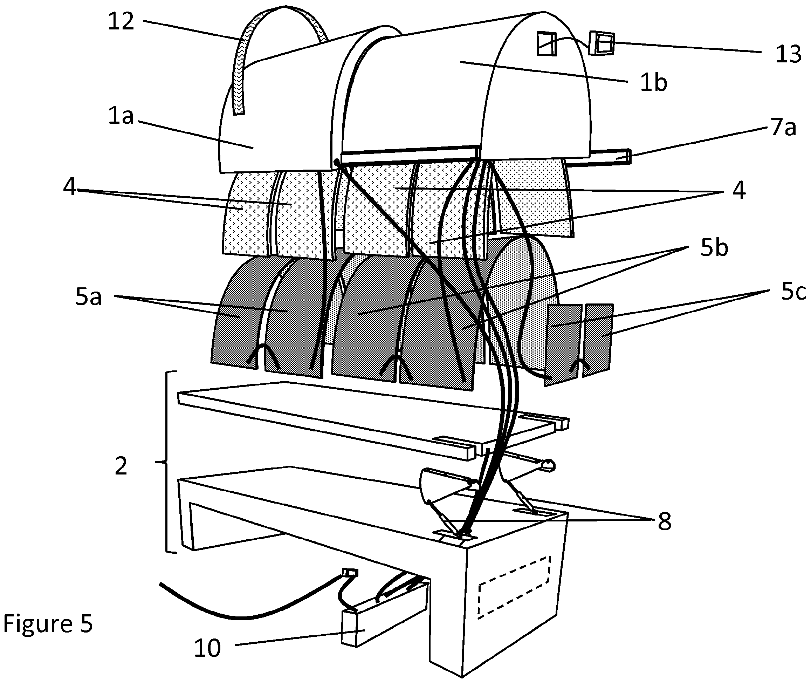

Dans la configuration illustrative et nullement limitative représentée sur la figure 5, la partie recouvrante (1) comprend une première portion et une deuxième portion. La première portion (1a) comprend une couche extérieure, deux couches (4) réflectrices et deux plaques en carbone (5a). La deuxième portion (1b) comprend également une couche extérieure, deux couches (4) réflectrices et deux plaques en carbone (5b). À une extrémité, la partie recouvrante est fermée par une plaque rigide sur laquelle sont fixées, par exemple deux plaques en carbone (5c). Le support (2) comprend également au moins une plaque en carbone.

Dans certains modes de réalisation, les plaques en carbone de la première portion sont alimentées indépendamment des autres plaques.

Dans certains modes de réalisation, les plaques de carbone et/ou les couches réflectrices sont fixées contre la couche extérieure par des moyens de fixation.

Dans certains modes de réalisation, les plaques en carbone de la deuxième portion sont alimentées indépendamment des autres plaques.

Dans certains modes de réalisation, les plaques en carbone du support (2) sont alimentées indépendamment des autres plaques.

Dans ces modes de réalisation où les plaques sont alimentées indépendamment les unes des autres, il est possible de moduler le rayonnement infrarouge selon les régions du volume compris entre la partie recouvrante et le support (2). Par exemple, l'alimentation des plaques en carbone de la première portion sont réglées de telle manière que le rayonnement infrarouge produit a une intensité moins importante que le rayonnement produit par les plaques en carbone de la deuxième portion.

Dans certains modes de réalisation, chaque portion et/ou le support comprend plusieurs plaques régulées indépendamment les unes des autres, de façon à fournir une modulation encore plus fine du rayonnement infrarouge, par exemple selon les zones à traiter dans le cas de la phlébologie. Cet agencement permet encore d'améliorer la fiabilité et la longévité du dispositif.

Indépendamment du contrôle de la température, du régulateur ou même de la forme du profil de la partie recouvrante, le dispositif d'irradiation comprend un système de relèvement de la partie recouvrante. La première portion (1a) ou la deuxième portion (1b) de la partie (1) recouvrante est fixée sur au moins le système de relèvement de la partie (1) recouvrante. Le système de relèvement de la partie (1) recouvrante comprend au moins un vérin (8) de poussée dont une extrémité est fixée sur le support (2) ou sur les pieds du support (2) et l'autre extrémité est fixée sur la première ou la deuxième portion (1b) de la partie (1) recouvrante de manière à ce que le vérin (8) puisse exercer une poussée tendant à lever une extrémité de la partie (1) recouvrante. L'autre extrémité de la partie (1) recouvrante est fixée sur le support (2) par l'intermédiaire d'une articulation autorisant le fait que l'axe (11) longitudinal de la partie (1) recouvrante puisse faire un angle non nul avec le plan du support (2). La force de poussée du ou des vérins (8) est déterminée en fonction de différentes position de la première portion (1a). Lorsque la première portion (1a) ne recouvre pas la deuxième portion (1b), la force de poussée du vérin (8) n'est pas suffisante pour soulever l'extrémité non fixée de la partie (1) recouvrante dont l'axe (11) longitudinal reste parallèle avec le plan du support (2). Lorsque la première portion (1a) recouvre la deuxième portion (1b), la force du vérin (8) est suffisante pour soulever l'extrémité fixée de la partie (1) recouvrante dont l'axe (11) longitudinal fait alors un angle non nul avec le plan du support (2). Un élément de butée permet de retenir la partie (1) recouvrante lorsqu'elle est soulevée par le ou les vérins (8) de limiter l'angle que fait l'axe (11) longitudinal de la partie (1) recouvrante et le plan du support (2).

Cet agencement présente l'avantage de faciliter l'ouverture du dispositif par l'utilisation lui-même ne poussant sur la première portion pour la faire coulisser et la première et la deuxième portion se soulèvent automatiquement sous l'action du vérin.

L'invention concerne donc éventuellement un dispositif comprenant un support (2) et une partie (1) recouvrante disposés selon un axe longitudinal du dispositif. La partie recouvrante comprend une première portion montée coulissante sur une deuxième portion fixée par un axe de pivotement sur le support. Le dispositif comprend un vérin monté entre la deuxième portion et le support (2) ou les pieds du support (2), la force de poussée du vérin étant adaptée pour que, d'une part, le poids des portions en position déployée empêche le soulèvement des deux portions, et, d'autre part, lorsque les portions sont escamotées, au moins partiellement, la force du vérin suffise à faire se relever automatiquement la partie recouvrante.

La présente description détaille différents modes de réalisation et configuration ou variantes en référence à des figures et/ou des caractéristiques techniques. L'homme du métier comprendra que les diverses caractéristiques techniques des divers modes ou configurations peuvent être combinées entre elles pour obtenir d'autres modes de réalisation et de configuration, à moins que l'inverse ne soit explicitement mentionné ou que ces caractéristiques techniques ne soient incompatibles. De même, une caractéristique technique d'un mode de réalisation ou d'une configuration peut être isolée des autres caractéristiques techniques de ce mode de réalisation à moins que l'inverse ne soit mentionné. Dans la présente description, de nombreux détails spécifiques sont fournis à titre illustratif et nullement limitatif, de façon à détailler précisément l'invention. L'homme de métier comprendra cependant que l'invention peut être réalisée en l'absence d'un ou plusieurs de ces détails spécifiques ou avec des variantes. À d'autres occasions, certains aspects ne sont pas détaillés de façon à éviter d'obscurcir et alourdir la présente description et l'homme de métier comprendra que des moyens divers et variés pourront être utilisés et que l'invention n'est pas limitée aux seuls exemples décrits.

Il doit être évident pour les personnes versées dans l'art que la présente invention permet des modes de réalisation sous de nombreuses autres formes spécifiques sans l'éloigner du domaine d'application de l'invention comme revendiqué. Par conséquent, les présents modes de réalisation doivent être considérés à titre d'illustration, mais peuvent être modifiés dans le domaine défini par la portée des revendications jointes, et l'invention ne doit pas être limitée aux détails donnés ci-dessus.

**Revendications**

1. Dispositif d'irradiation à infrarouge comprenant un support (2) apte à recevoir un utilisateur (9) et une partie (1) recouvrante apte à recouvrir l'utilisateur (9), la partie (1) recouvrante comprenant au moins une couche chauffante (5a, 5b, 5c) apte à émettre un rayonnement (6) infrarouge dans une plage de longueurs d'onde déterminée vers au moins une partie du volume situé entre la face interne de la couche chauffante (5a, 5b, 5c) et le support (2), ladite couche chauffante (5a, 5b, 5c) étant régulée par au moins un contrôleur (10) comprenant des moyens d'alimentation (16) fournissant la puissance nécessaire à l'émission d'infrarouge pendant une séance d'irradiation et au moins un contacteur, le dispositif étant **caractérisé en ce qu'**il comprend en outre au moins un programmateur (15) adapté à déterminer les paramètres de la séance d'irradiation, en définissant des pulsations au cours de la séance ; ces pulsations contrôlant, d'une part, l'alimentation de ladite couche chauffante (5a, 5b, 5c) à des fréquences variables, et d'autre part, le contacteur du dispositif, par exemple un relais statique synchrone (14) ou un autre type de contacteur qui délivre, à ladite couche chauffante (5a, 5b, 5c), la puissance fournie par les moyens d'alimentation (16) au cours des pulsations.

2. Dispositif selon la revendication 1, **caractérisé en ce que** le contacteur est un relais statique synchrone (14) qui ne délivre aucun courant à ladite couche chauffante (5a, 5b, 5c) en dehors des pulsations définies par le programmateur (15).

3. Dispositif selon une des revendications 1 et 2, **caractérisé en ce que** le programmateur (15) calcule le temps auquel les pulsations doivent être délivrées, ainsi que leur durée, en fonction de la quantité d'énergie à fournir, et donc de la température demandée.

4. Dispositif selon une des revendications 1 à 3, **caractérisé en ce que** le programmateur (15) comporte une mémoire stockant une pluralité de programmes prédéfinis correspondant à divers types de séances d'irradiation et définissant la durée de la séance d'irradiation et/ou la ou les température(s) à atteindre au cours de la séance.

5. Dispositif selon une des revendications précédentes, **caractérisé en ce qu'**il comporte au moins une sonde ou capteur (18) et le programmateur (15) calcule les pulsations en fonction d'informations de température provenant de ladite sonde ou dudit capteur (18).

6. Dispositif selon la revendication 5, **caractérisé en ce que** la dite au moins une sonde ou capteur (18, 18a, 18b, 18c) est au moins une sonde de température permettant au programmateur d'ajuster les pulses en fonction de la température obtenue dans le dispositif.

7. Dispositif selon une des revendications précédentes, **caractérisé en ce qu'**il est muni de moyens de sécurité électrique (17) pour protéger l'utilisateur d'éventuels problèmes dans les circuits électriques du dispositif.

8. Dispositif selon une des revendications précédentes, **caractérisé en ce que** la partie (1) recouvrante comprend en outre une couche (4) réflectrice de rayonnement (6) infrarouge.

9. Dispositif selon au moins une des revendications précédentes, **caractérisé en ce que** le support (2) comprend une deuxième couche chauffante apte à émettre un rayonnement dans au moins une partie du volume entre la face interne de la première couche chauffante et le support (2).

10. Dispositif selon au moins une des revendications précédentes, **caractérisé en ce qu'**une extrémité, selon l'axe longitudinal, de la partie (1) recouvrante comprend une troisième couche (5c) chauffante fermant la partie (1) recouvrante.

11. Dispositif selon au moins une des revendications précédentes, **caractérisé en ce que** la partie (1) recouvrante comprend au moins deux portions, la deuxième portion (1b) de la partie (1) recouvrante étant apte à coulisser par rapport à la première portion (1a) parallèlement à l'axe (11) longitudinal de sorte à s'escamoter dans ou sur la première portion (1a).

12. Dispositif selon la revendication 11, **caractérisé en ce qu'**il comporte, d'une part, un câblage d'alimentation de la première couche chauffante et, d'autre part, un guide pour permettre le coulissement des deux portions (1a, 1b) sans coincer et/ou endommager le câblage.

13. Dispositif selon au moins une des revendications précédentes, **caractérisé en ce que** la partie (1) recouvrante comprend une couche (3) extérieure protégeant l'intérieur de la partie (1) recouvrante.

**14.** Dispositif selon au moins une des revendications précédentes, **caractérisé en ce que** ladite (au moins une) couche chauffante comporte au moins une plaque en carbone.

**15.** Dispositif selon au moins une des revendications 8 à 14, **caractérisé en ce que** la couche (4) réflectrice comprend au moins une couche en aluminium.

**16.** Dispositif selon la revendication 13, **caractérisé en ce que** la couche (3) extérieure est en bois.

**17.** Dispositif selon au moins une des revendications précédentes, **caractérisé en ce que** le support (2) est en bois.

**18.** Dispositif selon au moins une des revendications précédentes, **caractérisé en ce que** la surface interne de la partie recouvrante est recouverte d'un textile.

**19.** Dispositif selon au moins une des revendications précédentes, **caractérisé en ce que** le dispositif d'irradiation comprend un système de relèvement de la partie recouvrante, la première portion (1a) ou la deuxième portion (1b) de la partie (1) recouvrante étant fixée sur au moins le système de relèvement de la partie (1) recouvrante, le système de relèvement de la partie (1) recouvrante comprenant au moins un vérin (8) de poussée dont une extrémité est fixée sur le support (2) ou sur les pieds du support (2) et l'autre extrémité est fixée sur la première ou la deuxième portion (1b) de la partie (1) recouvrante de manière à ce que le vérin (8) puisse exercer une poussée tendant à lever une extrémité de la partie (1) recouvrante, l'autre extrémité de la partie (1) recouvrante étant fixée sur le support (2) par l'intermédiaire d'une articulation autorisant le fait que l'axe (11) longitudinal de la partie (1) recouvrante puisse faire un angle non nul avec le plan du support (2).

**20.** Dispositif selon la revendication précédente, **caractérisé en ce qu'**il comporte un câblage d'alimentation de la couche chauffante (5a, 5b) qui passe à proximité de l'articulation de la partie recouvrante pour en faciliter le pivotement sans coincer et/ou endommager le câblage.

**Patentansprüche**

**1.** Infrarotbestrahlungsvorrichtung, die einen Ständer (2) umfasst, der dazu geeignet ist, einen Benutzer (9) und einen Umhüllungsteil (1), der dazu geeignet ist, den Benutzer (9) zu umhüllen, aufzunehmen, wobei der Umhüllungsteil (1) mindestens eine Heizschicht (5a, 5b, 5c) umfasst, die dazu geeignet ist, eine Infrarotstrahlung (6) in einem bestimmten Wellenlängenbereich in Richtung mindestens eines Teils des Rauminhalts, der sich zwischen der Innenseite der Heizschicht (5a, 5b, 5c) und dem Ständer (2) befindet, abzustrahlen, wobei die Heizschicht (5a, 5b, 5c) durch mindestens eine Steuerung (10) reguliert wird, die Versorgungsmittel (16) umfasst, die die zur Infrarotabstrahlung erforderliche Leistung während einer Bestrahlungssitzung zuführen, und mindestens ein Schütz,
wobei die Vorrichtung **dadurch gekennzeichnet ist, dass** sie außerdem mindestens einen Scheduler (15) umfasst, der dazu geeignet ist, die Parameter der Bestrahlungssitzung durch Definieren von Pulsationen im Verlauf der Sitzung zu bestimmen; wobei diese Pulsationen einerseits die Versorgung der Heizschicht (5a, 5b, 5c) mit variablen Frequenzen und andererseits das Schütz der Vorrichtung, beispielsweise ein statisches Synchronrelais (14) oder ein anderer Schütztyp, der die Leistung, die von den Versorgungsmitteln (16) zugeführt wird, im Verlauf von Pulsationen an die Heizschicht (5a, 5b, 5c) abgibt, steuert.

**2.** Vorrichtung nach Anspruch 1, **dadurch gekennzeichnet, dass** das Schütz ein statisches Synchronrelais (14) ist, das abgesehen von Pulsationen, die von dem Scheduler (15) definiert werden, keinen Strom an die Heizschicht (5a, 5b, 5c) abgibt.

**3.** Vorrichtung nach einem der Ansprüche 1 und 2, **dadurch gekennzeichnet, dass** der Scheduler (15) die Zeit, zu der die Pulsationen abgegeben werden müssen, sowie deren Dauer in Abhängigkeit von der abzugebenden Energiemenge und folglich der geforderten Temperatur berechnet.

**4.** Vorrichtung nach einem der Ansprüche 1 bis 3, **dadurch gekennzeichnet, dass** der Scheduler (15) einen Speicher umfasst, der mehrere vordefinierte Programme speichert, die verschiedenen Typen von Bestrahlungssitzungen entsprechen und die Dauer der Bestrahlungssitzung und/oder die im Verlauf der Sitzung zu erreichende Temperatur oder die im Verlauf der Sitzung zu erreichenden Temperaturen definieren.

**5.** Vorrichtung nach einem der vorhergehenden Ansprüche, **dadurch gekennzeichnet, dass** sie mindestens eine Sonde oder einen Sensor (18) umfasst und der Scheduler (15) die Pulsationen in Abhängigkeit von Temperaturinformationen, die von der Sonde oder dem Sensor (18) stammen, berechnet.

**6.** Vorrichtung nach Anspruch 5, **dadurch gekennzeichnet, dass** die mindestens eine Sonde oder der mindestens eine Sensor (18, 18a, 18b, 18c) mindestens eine Temperatursonde ist, die dem Scheduler ermöglicht, die Pulse in Abhängigkeit von der Temperatur, die in der Vorrichtung erlangt wird, zu justieren.

7. Vorrichtung nach einem der vorhergehenden Ansprüche, **dadurch gekennzeichnet, dass** sie mit elektrischen Sicherheitsmitteln (17) zum Schützen des Benutzers vor eventuellen Problemen in den elektrischen Stromkreisen der Vorrichtung ausgestattet ist.

8. Vorrichtung nach einem der vorhergehenden Ansprüche, **dadurch gekennzeichnet, dass** der Umhüllungsteil (1) außerdem eine Infrarotstrahlung (6) reflektierende Schicht (4) umfasst.

9. Vorrichtung nach mindestens einem der vorhergehenden Ansprüche, **dadurch gekennzeichnet, dass** der Ständer (2) eine zweite Heizschicht umfasst, die dazu geeignet ist, eine Strahlung in mindestens einem Teil des Rauminhalts zwischen der Innenseite der ersten Heizschicht und dem Ständer (2) abzustrahlen.

10. Vorrichtung nach mindestens einem der vorhergehenden Ansprüche, **dadurch gekennzeichnet, dass** ein Ende des Umhüllungsteils (1) entlang der Längsachse eine dritte Heizschicht (5c) umfasst, die den Umhüllungsteil (1) schließt.

11. Vorrichtung nach mindestens einem der vorhergehenden Ansprüche, **dadurch gekennzeichnet, dass** der Umhüllungsteil (1) mindestens zwei Abschnitte umfasst, wobei der zweite Abschnitt (1b) des Umhüllungsteils (1) dazu geeignet ist, in Bezug auf den ersten Abschnitt (1a) parallel zur Längsachse (1 1) derart zu gleiten, dass er in oder auf dem ersten Abschnitt (1a) eingefahren wird.

12. Vorrichtung nach Anspruch 11, **dadurch gekennzeichnet, dass** sie einerseits eine Versorgungsverdrahtung der ersten Heizschicht und andererseits eine Führung zum Ermöglichen des Gleitens von zwei Abschnitten (1a, 1b) ohne Einklemmen und/oder Beschädigen der Verdrahtung umfasst.

13. Vorrichtung nach mindestens einem der vorhergehenden Ansprüche, **dadurch gekennzeichnet, dass** der Umhüllungsteil (1) eine äußere Schicht (3) umfasst, die das Innere des Umhüllungsteils (1) schützt.

14. Vorrichtung nach mindestens einem der vorhergehenden Ansprüche, **dadurch gekennzeichnet, dass** die (mindestens eine) Heizschicht mindestens eine Platte aus Kohlenstoff umfasst.

15. Vorrichtung nach mindestens einem der Ansprüche 8 bis 14, **dadurch gekennzeichnet, dass** die reflektierende Schicht (4) mindestens eine Schicht aus Aluminium umfasst.

16. Vorrichtung nach Anspruch 13, **dadurch gekennzeichnet, dass** die äußere Schicht (3) aus Holz ist.

17. Vorrichtung nach mindestens einem der vorhergehenden Ansprüche, **dadurch gekennzeichnet, dass** der Ständer (2) aus Holz ist.

18. Vorrichtung nach mindestens einem der vorhergehenden Ansprüche, **dadurch gekennzeichnet, dass** die Innenfläche des Umhüllungsteils mit einem Gewebe bedeckt ist.

19. Vorrichtung nach mindestens einem der vorhergehenden Ansprüche, **dadurch gekennzeichnet, dass** die Bestrahlungsvorrichtung ein System zum Anheben des Umhüllungsteils umfasst, wobei der erste Abschnitt (1a) oder der zweite Abschnitt (1b) des Umhüllungsteils (1) an mindestens dem System zum Anheben des Umhüllungsteils (1) angebracht ist, wobei das System zum Anheben des Umhüllungsteils (1) mindestens einen Hubzylinder (8) umfasst, bei dem ein Ende an dem Ständer (2) oder an den Füßen des Ständers (2) angebracht ist und das andere Ende derart an dem ersten oder dem zweiten Abschnitt (1b) des Umhüllungsteils (1) angebracht ist, dass der Hubzylinder (8) eine Hubkraft ausübt, die dazu neigt, ein Ende des Umhüllungsteils (1) hochzuheben, wobei das andere Ende des Umhüllungsteils (1) an dem Ständer (2) mittels eines Gelenks angebracht ist, das zulässt, dass die Längsachse (11) des Umhüllungsteils (1) einen Nicht-Null-Winkel mit der Ebene des Ständers (2) bilden kann.

20. Vorrichtung nach dem vorhergehenden Anspruch, **dadurch gekennzeichnet, dass** sie eine Versorgungsverdrahtung der Heizschicht (5a, 5b) umfasst, die nahe des Gelenks des Umhüllungsteils verläuft, um das Schwenken ohne Einklemmen und/oder Beschädigen der Verdrahtung zu erleichtern.

## Claims

1. Infrared irradiation apparatus comprising a support (2) capable of receiving a user (9) and a covering part (1) capable of covering the user (9), the covering part (1) comprising at least one heating layer (5a, 5b, 5c) capable of emitting infrared radiation (6) in a given wavelength range towards at least a part of the volume situated between the inner face of the heating layer (5a, 5b, 5c) and the support (2), said heating layer (5a, 5b, 5c) being regulated by at least one controller (10) comprising supply means (16) supplying the power necessary for infrared emission during an irradiation session and at least one contactor,

the apparatus being **characterised in that** it further comprises at least one programmer (15) adapted to determine the parameters of the irradiation session, by defining pulsations during the session; these pulsations controlling, on the one hand, the supply to said heating layer (5a, 5b, 5c) at variable frequencies, and, on the other hand, the contactor of the apparatus, for example a synchronous static relay (14) or other type of contactor which delivers, to said heating layer (5a, 5b, 5c), the power supplied by the supply means (16) during the pulsations.

2. Apparatus according to claim 1, **characterised in that** the contactor is a synchronous static relay (14) which does not deliver any current to said heating layer (5a, 5b, 5c) outside the pulsations defined by the programmer (15).

3. Apparatus according to one of claims 1 and 2, **characterised in that** the programmer (15) calculates the time at which the pulsations must be delivered, as well as their duration, as a function of the quantity of energy to be supplied and therefore the requested temperature.

4. Apparatus according to one of claims 1 to 3, **characterised in that** the programmer (15) comprises a memory storing a plurality of predefined programs corresponding to various types of irradiation sessions and defining the duration of the irradiation session and/or the temperature or temperatures to be reached during the session.

5. Apparatus according to one of the preceding claims, **characterised in that** it comprises at least one probe or sensor (18) and the programmer (15) calculates the pulsations as a function of temperature information from said probe or sensor (18).

6. Apparatus according to claim 5, **characterised in that** said at least one probe or sensor (18, 18a, 18b, 18c) is at least one temperature probe allowing the programmer to adjust the pulses as a function of the temperature obtained in the apparatus.

7. Apparatus according to one of the preceding claims, **characterised in that** it is provided with electrical safety means (17) in order to protect the user from any problems in the electrical circuits of the apparatus.

8. Apparatus according to one of the preceding claims, **characterised in that** the covering part (1) further comprises a layer (4) which reflects infrared radiation (6).

9. Apparatus according to at least one of the preceding claims, **characterised in that** the support (2) comprises a second heating layer capable of emitting radiation in at least a part of the volume between the inner face of the first heating layer and the support (2).

10. Apparatus according to at least one of the preceding claims, **characterised in that** one end, along the longitudinal axis, of the covering part (1) comprises a third heating layer (5c) which closes the covering part (1).

11. Apparatus according to at least one of the preceding claims, **characterised in that** the covering part (1) comprises at least two portions, the second portion (1b) of the covering part (1) being capable of sliding relative to the first portion (1a) parallel to the longitudinal axis (11) so as to retract into or onto the first portion (1a).

12. Apparatus according to claim 11, **characterised in that** it comprises, on the one hand, supply wiring for the first heating layer and, on the other hand, a guide to allow sliding of the two portions (1a, 1b) without pinching and/or damaging the wiring.

13. Apparatus according to at least one of the preceding claims, **characterised in that** the covering part (1) comprises an outer layer (3) protecting the interior of the covering part (1).

14. Apparatus according to at least one of the preceding claims, **characterised in that** said (at least one) heating layer comprises at least one carbon plate.

15. Apparatus according to at least one of claims 8 to 14, **characterised in that** the reflective layer (4) comprises at least one aluminium layer.

16. Apparatus according to claim 13, **characterised in that** the outer layer (3) is made of wood.

17. Apparatus according to at least one of the preceding claims, **characterised in that** the support (2) is made of wood.

18. Apparatus according to at least one of the preceding claims, **characterised in that** the inner surface of the covering part is covered with a textile.

19. Apparatus according to at least one of the preceding claims, **characterised in that** the irradiation apparatus comprises a system for lifting the covering part, the first portion (1a) or the second portion (1b) of the covering part (1) being fixed to at least the system for lifting the covering part (1), the system for lifting the covering part (1) comprising at least one thrust actuator (8) of which one end is fixed to the support (2) or to the legs of the support (2) and the other end is fixed to the first or second portion (1b) of the covering part (1) in such a way that the actuator (8) can exert a thrust which leads to lifting one end of the covering part (1), the other end of the covering part (1) being fixed to the support (2) by means of a hinge joint allowing the longitudinal axis (11) of the covering part (1) to form a non-zero angle with the plane of the support (2).

20. Apparatus according to the preceding claim, **characterised in that** it comprises supply wiring for the heating layer (5a, 5b) which passes close to the hinge joint of the covering part in order to facilitate pivoting thereof without pinching and/or damaging the wiring.

Figure 1

1a

1b

1a

11

7b

2

7a

8

Figure 2

EP 3 060 300 B1

Figure 3

Figure 4

EP 3 060 300 B1

Figure 5

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

• US 2002183814 A1

**Littérature non-brevet citée dans la description**

• **MARTINE LAROCHE.** Rôle des ondes de surface dans la modification des propriétés radiatives de matériaux microstructurés. Application à la conception de sources infrarouges et à l'effet thermophotovoltaïque. *École centrale, Paris,* 2005